# EUROPEAN PATENT APPLICATION

(11) **EP 0 722 773 A1**
(43) Date of publication of application: **24.07.1996**
(21) Application number: 96100242.5
(22) Date of filing: 10.01.1996
(51) Int. Cl.: B01J 20/04, B01D 15/08

(54) **Adsorbent for activated TGF-beta1, method for detecting activated TGF-beta1, and method for detecting cancer**

(30) Priority: 18.01.1995 JP 5761/95
(71) Applicant: MITSUBISHI MATERIALS CORPORATION, Chiyoda-ku, Tokyo (JP)
(72) Inventor: Watanabe, Etsuko, c/o Sohgou-Kenkyusho, Omiya-shi, Saitama-ken (JP)
(74) Representative: Türk, Gille, Hrabal, Leifert

(57) **Abstract**

Disclosed is an adsorbent for activated TGF-β1 comprising, as the active ingredient, an OH-carbonated hydroxyapatite to be obtained by partly or wholly substituting the OH groups in hydroxyapatite with CO₃ groups. The adsorbent thus selectively adsorbs activated TGF-β1. A fluid containing activated TGF-β1 is brought into contact with the absorbent to make the activated TGF-β1 selectively adsorbed by the adsorbent, then the thus-adsorbed, activated TGF-β1 is eluted with a buffer, and the concentration of the activated TGF-β1 in the fluid is determined. According to this detecting method, even a minor amount of activated TGF-β1 in a sample can be detected. The adsorbent is applicable to detection of cancer, by which the concentration of activated TGF-β1 in a body fluid sample can be directly determined.

## Description

### FIELD OF THE INVENTION

The present invention relates to an adsorbent for activated TGF-β1, which is known to be the essential substance that causes the autosecretory propagation of cancer cells, a method for detecting activated TGF-β1, and a method for detecting cancer by utilizing the detection of activated TGF-β1.

### BACKGROUND OF THE INVENTION

Various medicines for curing cancer have heretofore been studied and developed. However, curing of cancer involves a problem in immunosuppression to be caused by chemotherapy or by cancer itself. Diagnosis of cancer, including detection of cancer in its early stages prior to curing it and detection of metastasis or relapse in cancer in its early stages during or after its curing, is also an important factor in curing cancer. To detect cancer, in general, a method has heretofore been employed of taking out the affected tissue followed by directly examining the morphology of the cells in the tissue. As the case may be, therefore, it is often difficult according to the method to judge as to whether the affected tissue has a benign or malignant tumor before the surgical operation. On the other hand, for specific immunological serodiagnosis of cancer, a method has been proposed of measuring the cancer-specific glycoprotein in a patient as the marker for detection of cancer (for example, see Japanese Patent Publication No. 62-100661).

The tumor markers that have heretofore been employed for detection of cancer include various physiologically-active substances existing in living organisms, and there is some relationship between the content of the substance and the estimated disease. In fact, however, many of such substances are often detected in normal cells or are hardly differentiated from those derived from normal cells in qualitative assay. In particular, cancer markers which are qualitatively and quantitatively effective for detection of cancer have not been known as yet at present.

On the other hand, inactive TGF-β1 exists in the blood of normal persons. When the inactive TGF-β1 is brought into contact with cancer cells, it is activated and promotes the auto-secretion and then propagation of the cancer cells. In addition, it is known that cancer cells produce activated TGF-β1 and inactive TGF-β1 is activated by enzyme or the like, whereby the concentration of the activated TGF-β1 in blood is increased and the immunity to cancer is suppressed. At present, however, it is not easy to measure the concentration of a minor amount of TGF-β1 in body fluids such as blood, etc., and it is further more difficult to selectively determine the concentration of activated TGF-β1 therein.

As the method for detecting cancer by measuring the concentration of TGF-β1 in blood, an ELISA (enzyme-linked immunosorbent assay) method (such as that described in Japanese Patent Laid-Open No. 5-336990) has now been put to practical use.

However, the ELISA method have various problems in that the operation for the method is complicated and needs at least 3 days, that the costs for the method are high, and that TGF-β1 in a sample is wholly activated in the pretreatment step so that the data derived from the method are not always accurate.

On the other hand, a carbonated hydroxyapatite (Ca₁₀(PO₄)₆(CO₃)ₓ(OH)₂₋₂ₓ) to be obtained by substituting a part of the (OH) sites in hydroxyapatite (Ca₁₀(PO₄)₆(OH)₂) with (CO₃) is known, and this is referred to as an OH-carbonated hydroxyapatite. It has heretofore been unknown that the OH-carbonated hydroxyapatite is usable as an adsorbent for TGF-β1.

### SUMMARY OF THE INVENTION

One object of the present invention is to provide an adsorbent capable of selectively adsorbing activated TGF-β1 and an effective method for detecting activated TGF-β1 by the use of this adsorbent.

Another object of the present invention is to provide a highly-accurate method for detecting cancer by measuring activated TGF-β1, which is the essential substance that causes the autosecretory propagation of cancer cells.

According to the present invention, there is provided an adsorbent (hereinafter referred to as adsorbent A) for activated TGF-β1 that comprises, as the active ingredient, an OH-carbonated hydroxyapatite to be obtained by partly or wholly substituting the OH groups in hydroxyapatite with CO₃ groups.

There is also provided according to the present invention a method for detecting activated TGF-β1, wherein a fluid containing activated TGF-β1 is brought into contact with the adsorbent A to make the activated TGF-β1 selectively adsorbed by the adsorbent A, then the thus-adsorbed, activated TGF-β1 is eluted with a buffer, and the concentration of the activated TGF-β1 in the fluid is determined.

There is further provided according to the present invention a method for detecting cancer, wherein a body fluid is brought into contact with the adsorbent A to make activated TGF-β1 that exists in the body fluid selectively adsorbed by the adsorbent A, then the thus-adsorbed, activated TGF-β1 is eluted with a buffer, and the concentration of the activated TGF-β1 in the body fluid is determined.

### BRIEF DESCRIPTION OF THE DRAWINGS

Fig. 1 is a chromatogram of the eluate obtained in Example 1 and detected with an ultraviolet detector (at 280 nm).

Fig. 2(a), Fig. 2(b) and Fig. 2(c) are chromatograms of the eluates obtained in Example 2 from three blood samples of normal panelists each comprising serum and plasma and detected with an ultraviolet detector (at 280 nm).

Fig. 3(a), 3 (b) and 3(c) are chromatograms of the eluates obtained in Example 2 from three serum samples of cancer patients and detected with an ultraviolet detector (at 280 nm).

Fig. 4 is a chromatogram of the eluate obtained from the same standard sample as that used in Example 1, by the use of a synthetic hydroxyapatite as produced in Comparative Example 1, and detected with an ultraviolet detector (at 280 nm).

Fig. 5(a), Fig. 5(b) and Fig. 5(c) are chromatograms of the eluates obtained in Comparative Example 1 from three blood samples of normal panelists each comprising serum and plasma and detected with an ultraviolet detector (at 280 nm).

Fig. 6(a), 6 (b) and 6(c) are chromatograms of the eluates obtained in Comparative Example 1 from three serum samples of cancer patients and detected with an ultraviolet detector (at 280 nm).

### DETAILED DESCRIPTION OF THE INVENTION

The present invention is described in more detail hereinunder.

The adsorbent A of the present invention comprises, as the active ingredient, an OH-carbonated hydroxyapatite (Ca₁₀(PO₄)₆(CO₃)ₓ(OH)₂₋₂ₓ) to be obtained by partly or wholly substituting the OH groups in hydroxyapatite with CO₃ groups. Concretely, it is especially preferred that the CO₃ content of the OH-carbonated hydroxyapatite for use in the present invention is 2.0 % by weight or more relative to the whole weight of the OH-carbonated hydroxyapatite itself.

The OH-carbonated hydroxyapatite selectively adsorbs only activated TGF-β1, and it is considered that the essential factor in the adsorption is the difference in the fine three-dimensional structure between the surface of the OH-carbonated hydroxyapatite which is extremely porous and the local surface of the molecule of activated TGF-β1 and that the peculiar adsorption will be realized on the basis of the difference in the intermolecular local structure.

Regarding its shape, it is desirable that the OH-carbonated hydroxyapatite is granular or powdery so that it can easily be filled in columns, etc., and it is desirable that its mean particle size falls between 3 µm and 20 µm. If its mean particle size is less than 3 µm and if a packing material comprising the OH-carbonated hydroxyapatite having such a small mean particle size is filled in a column through which a fluid is passed, the pressure loss in the column is too large. If, however, its mean particle size is more than 20 µm, the surface area per unit volume of the OH-carbonated hydroxyapatite is too small with the result that the separability thereof is unfavorably lowered.

To prepare the above-mentioned OH-carbonated hydroxyapatite for use in the present invention, employable are known methods, for example, including a method of heating hydroxyapatite in carbon dioxide at a normal pressure at about 1000°C. To make it granular or powdery, it may be granulated or powdered by any of known spray-drying granulation, grinding granulation, rotary granulation, etc.

To actually use the adsorbent A of the present invention, for example, employable is a method of filling the adsorbent A in a cylindrical container such as a column or the like followed by introducing thereinto a fluid containing activated TGF-β1 and passing the fluid through the container, by which only the activated TGF-β1 is made selectively adsorbed by the adsorbent A in the container.

To detect activated TGF-β1 according to the method of the present invention, a fluid containing activated TGF-β1 is first brought into contact with the adsorbent A whereby the activated TGF-β1 is made selectively adsorbed by the adsorbent A. The adsorption can be conducted, for example, according the above-mentioned method of using a column or the like, or a method of filling the adsorbent A in a centrifuger at its intercentrifugal center followed by introducing a fluid containing activated TGF-β1 thereinto while driving the centrifuger.

In the next step of the detecting method of the present invention, the thus-adsorbed, activated TGF-β1 is released from the adsorbent A by elution with a buffer. As the buffer, usable is a phosphate buffer such as a KH₃PO₄ buffer or the like. The concentration of the buffer is not specifically defined, provided that it is enough to make the activated TGF-β1 released from the adsorbent A by the elution. Concretely, however, it is desirable that the phosphate concentration in the buffer is from 200 to 350 mM.

To conduct the elution, for example, employable is a method of introducing a buffer such as that mentioned above into the column or the like cylindrical container having therein the adsorbent A that has adsorbed activated TGF-β1 and passing the buffer therethrough. If desired, the concentration of the buffer may be varied for linear gradient elution, in which the resulting eluate fractions are separately recovered and their concentrations are measured in the manner that is mentioned hereinunder whereby the definite concentration of the activated TGF-β1 in the activated TGF-β1-containing fluid can be determined accurately.

Subsequently, according to the detecting method of the present invention, the concentration of the activated TGF-β1 in the buffer as recovered in the aforementioned step may be measured to determine the concentration of the activated TGF-β1 in the activated TGF-β1-containing fluid. To measure the concentration of the activated TGF-β1 in the buffer, for example, employable are UV absorptiometry, colorimetry using a protein-coloring reagent, etc.

The method of the present invention for detecting cancer follows the above-mentioned method for detecting activated TGF-β1. The two are the same except that a body fluid sample is brought into contact with the adsorbent A in the former in place of the activated TGF-β1-containing fluid in the latter.

The body fluid sample includes blood samples and others as collected from animals including human beings. Preferably, a sample comprising serum and/or plasma to be obtained by removing albumin from a blood sample is brought into contact with the adsorbent A.

As has been described in detail hereinabove, since the adsorbent of the present invention comprises, as the active ingredient, an OH-carbonated hydroxyapatite, it can selectively adsorb only activated TGF-β1. In addition, since the adsorbent comprising such an OH-carbonated hydroxyapatite is used to adsorb activated TGF-β1 and the activated TGF-β1 thus adsorbed by the adsorbent is released therefrom by elution with a buffer in accordance with the detecting method of the present invention, even a minor concentration of activated TGF-β1 in a fluid sample can be accurately and effectively determined. Moreover, according to the other method of the present invention for detecting cancer, which follows the above-mentioned detecting method, the concentration of activated TGF-β1 in a body fluid sample that has been collected from an animal can be determined directly, easily and accurately without requiring any pretreatment of the sample for conventional ELISA, within a short period of time.

The present invention is described in- more detail by means of the following examples and comparative examples, which, however, are not intended to restrict the scope of the present invention.

### Example 1:

2 g of OH-carbonated hydroxyapatite which had been derived from hydroxyapatite by substituting OH groups in the hydroxyapatite with 3.4 % by weight of CO₃ groups (relative to the total weight of hydroxyapatite) and which had a mean particle size of 12.3 µm was, as an adsorbent for activated TGF-β1, was suspended in 15 ml of pure water, and the resulting suspension was introduced into a stainless column of 150 mm x 4 mmφ by a wet process at 2000 kg/cm² and at a flow rate of about 4.5 ml/min.

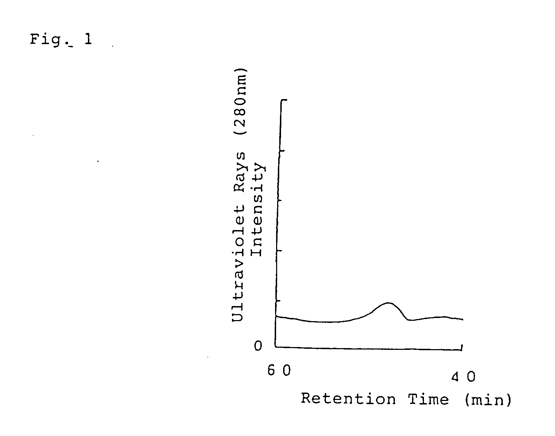

As the standard sample prepared was a solution of activated TGF-β1 by dissolving 1 µg of activated TGF-β1 in 30 µl of 5 mM HCl. 20 µl of the solution of activated TGF-β1 was injected into the column filled with the above-mentioned OH-carbonated hydroxyapatite through a sample injector, and then pure water was introduced thereinto at a rate of 0.4 ml/min. After fixation for 20 minutes, KH₃PO₄ (pH 7.4) buffers, 6 ml each, having a gradient concentration of from 0 M to 0.35 M was introduced into the column, with which the adsorbed substance was released by linear gradient elution. The substance thus released in each eluate was quantitatively detected with an ultraviolet (UV) detector at 280 nm. The results are shown in Fig. 1.

Fig. 1 shows the result of the detection for the standard sample containing only activated TGF-β1, in which the peak appeared indicates the activated TGF-β1 in the sample.

### Example 2:

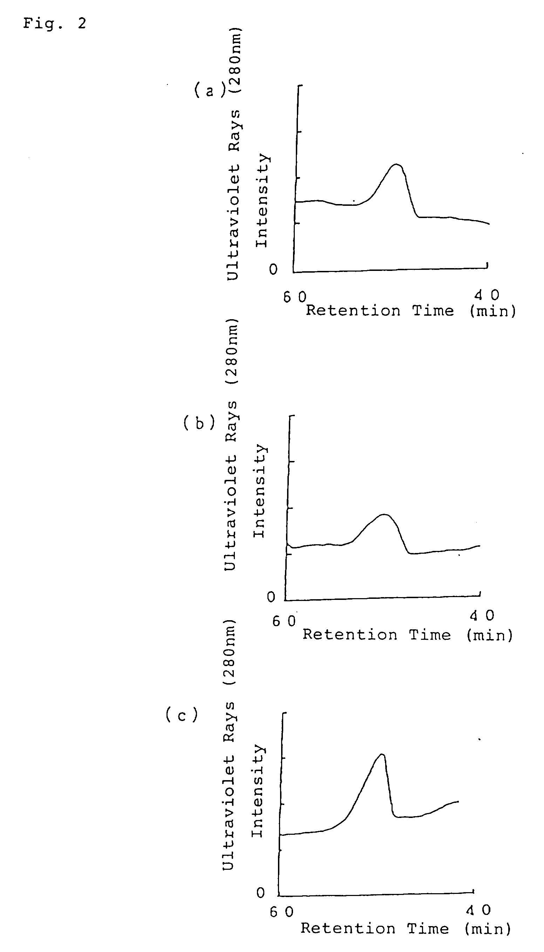

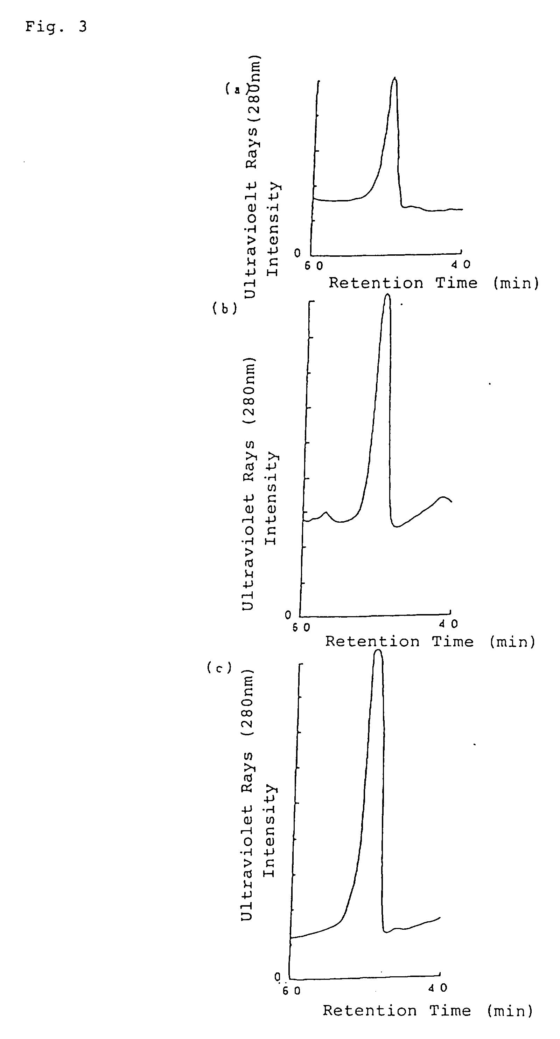

Three blood samples each comprising serum and plasma that had been collected from different normal persons and three serum samples collected from different cancer patients were filtered through an ultrafilter of 30 kDa and tested, 20 µl of each sample was injected into the column that had been filled with the OH-carbonated hydroxyapatite as prepared in Example 1, through a sample injector. Next, in the same manner as in Example 1, the substance as released in each eluate was quantitatively detected. The results are shown in Fig. 2(a) to Fig. 2(c) and Fig. 3(a) to Fig. 3(c).

The position of the peak appeared in each of Fig. 2(a) to Fig. 2(c) and Fig. 3(a) to Fig. 3(c) corresponds to that of the peak appeared in Fig. 1. Thus, it has been confirmed that the peak in these indicates the activated TGF-β1 in each sample. Comparing the peaks in Figs. 2(a) to 2(c) with those in Figs. 3(a) to 3(c), it is known that the peaks in Figs. 3(a) to 3(c) are from about 6 to about 10 times those in Figs. 2(a) to 2(c). Thus, it has been confirmed that the peaks detected in the serum samples collected from cancer patients are larger than those detected in the blood (serum and plasma) samples collected from normal persons. From these results, it is known that the adsorbent of the present invention is substantially useful for detecting cancer.

### Comparative Example 1:

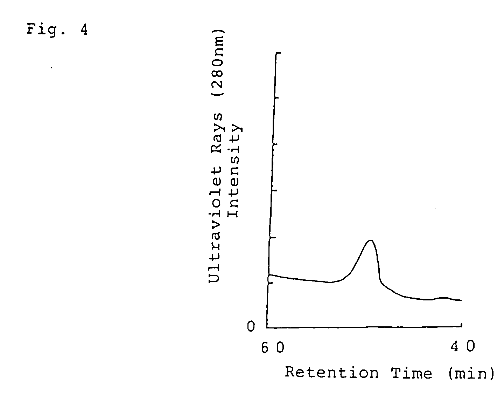

A hydroxyapatite powder having a mean particle size of 12.3 µm that had been prepared by a wet process and granulated by a dry process was calcined at 900°C to obtain a hydroxyapatite adsorbent. The thus-obtained adsorbent was filled in a column in the same manner as in Example 1, and 20 µl of each of the same standard sample as that in Example 1 and the same blood (serum and plasma) samples collected from normal persons and the same serum samples from cancer patients as those in Example 2 was injected into the column and treated in the same manner as in Example 1. The results obtained are shown in Fig. 4, Figs. 5(a) to 5(c) and Figs. 6(a) to 6(c).

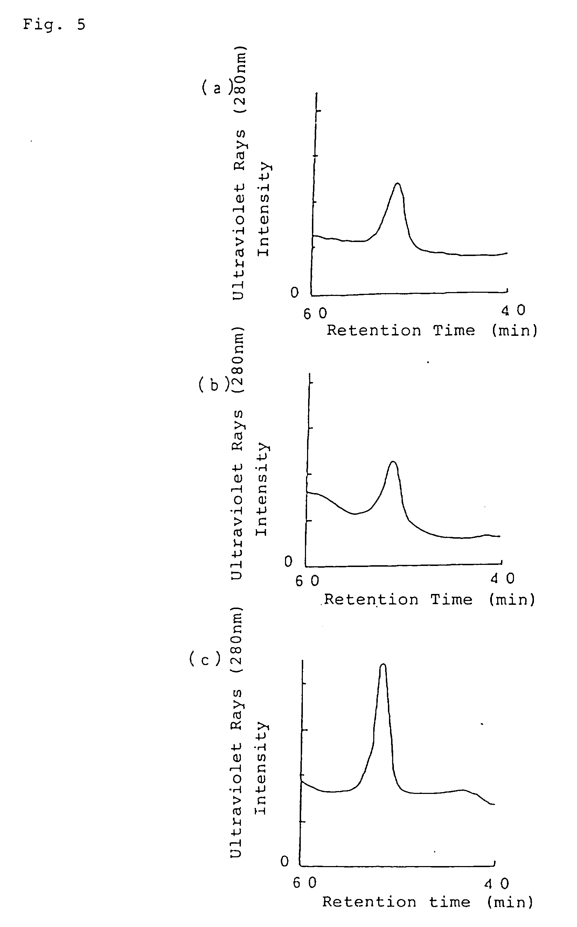

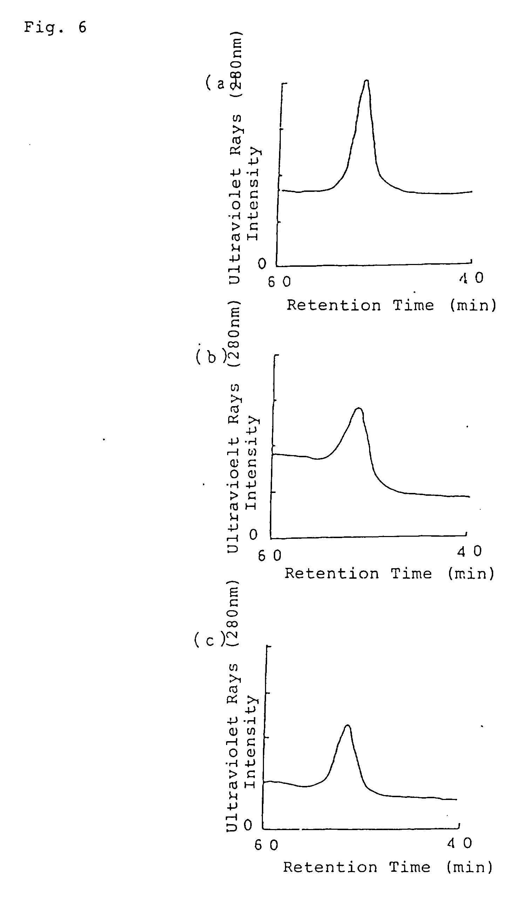

From Fig. 4, it is known that the hydroxyapatite adsorbent can also be used for detecting activated TGF-β1. However, when the results in Figs. 5(a) to 5(c) and Figs. 6(a) to 6(c) are compared with those in Figs. 2(a) to 2(c) and Figs. 3(a) to 3(c), there appears no difference in the peak between the samples from normal persons and those from cancer patients in the former while the latter gave the distinct difference in the peak therebetween as in Example 2. From these results, it is known that, being different from the OH-carbonated hydroxyapatite prepared in Example 1, the hydroxyapatite prepared and employed herein cannot quantitatively determine activated TGF-β1 and therefore cannot be used for accurately detecting cancer.

While the invention has been described in detail and with reference to specific embodiments thereof, it will be apparent to one skilled in the art that various changes and modifications can be made therein without departing from the spirit and scope thereof.

## Claims

1. An adsorbent for activated TGF-β1 comprising, as the active ingredient, an OH-carbonated hydroxyapatite to be obtained by partly or wholly substituting the OH groups in hydroxyapatite with CO₃ groups.

2. A method for detecting activated TGF-β1, wherein a fluid containing activated TGF-β1 is brought into contact with an absorbent as set forth in claim 1 to make the activated TGF-β1 selectively adsorbed by the adsorbent, then the thus-adsorbed, activated TGF-β1 is eluted with a buffer, and the concentration of the activated TGF-β1 in the fluid is determined.

3. A method for detecting cancer, wherein a body fluid is brought into contact with an adsorbent as set forth in claim 1 to make activated TGF-β1 that exists in the body fluid selectively adsorbed by the adsorbent, then the thus-adsorbed, activated TGF-β1 is eluted with a buffer, and the concentration of the activated TGF-β1 in the body fluid is determined.
